(19) **Europäisches Patentamt / European Patent Office / Office européen des brevets**

(11) **EP 2 953 700 B1**

(12) **EUROPEAN PATENT SPECIFICATION**

(45) Date of publication and mention
of the grant of the patent:
**07.04.2021 Bulletin 2021/14**

(21) Application number: **14749278.9**

(22) Date of filing: **07.02.2014**

(51) Int Cl.:
**B01D 17/04** (2006.01)  **C02F 1/36** (2006.01)
**C02F 1/40** (2006.01)

(86) International application number:
**PCT/US2014/015382**

(87) International publication number:
**WO 2014/124306 (14.08.2014 Gazette 2014/33)**

(54) **BIOREACTOR USING ACOUSTIC STANDING WAVES**

BIOREAKTOR MIT AKUSTISCHEN STEHWELLEN

BIORÉACTEUR UTILISANT DES ONDES ACOUSTIQUES STATIONNAIRES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **07.02.2013 US 201361761717 P
15.03.2013 US 201313844754
13.09.2013 US 201314026413**

(43) Date of publication of application:
**16.12.2015 Bulletin 2015/51**

(73) Proprietor: **Flodesign Sonics Inc.
Wilbraham, MA 01095 (US)**

(72) Inventors:
• **LIPKENS, Bart**
**Hampden, MA 01036 (US)**
• **MASI, Louis**
**Wilbraham, MA 01095 (US)**
• **KOWALSKI III, Stanley**
**Wilbraham, MA 01095 (US)**
• **PRESZ, Walter M. jr.**
**Wilbraham, MA 01095 (US)**
• **DIONNE, Jason**
**Simsbury, CT 06070 (US)**
• **DUTRA, Brian**
**Wilbraham, MA 01095 (US)**
• **MERCADO, Ari**
**Wilbraham, MA 01095 (US)**

• **KENNEDY III, Thomas**
**Wilbraham, MA 01095 (US)**
• **MARTIN, Arthur**
**Wilbraham, MA 01095 (US)**

(74) Representative: **Kador & Partner PartG mbB
Corneliusstraße 15
80469 München (DE)**

(56) References cited:
**WO-A1-2014/014941      US-A- 3 555 311
US-A- 5 443 985        US-A- 5 452 267
US-A1- 2011 123 392    US-A1- 2012 001 032
US-A1- 2012 329 122    US-A1- 2014 011 240
US-A1- 2014 011 240**

• **BENES E ET AL: "Ultrasonic separation of
suspended particles", 2001 IEEE ULTRASONICS
SYMPOSIUM PROCEEDINGS. ATLANTA, GA,
OCT. 7 - 10, 2001; [IEEE ULTRASONICS
SYMPOSIUM PROCEEDINGS], NEW YORK, NY :
IEEE, US, vol. 1, 7 October 2001 (2001-10-07),
pages 649-659, XP010584603, DOI:
10.1109/ULTSYM.2001.991812 ISBN:
978-0-7803-7177-4**
• **BENES ET AL.: 'Ultrasonic Separation of
Suspended Particles.' PROC. OF THE 2001 IEEE
INTEMATIONAL ULTRASONICS SYMPOSIUM, A
JOINT MEETING WITH THE WORLD CONGRESS
ON ULTRASONICS 07 October 2001, ATLANTA,
GEORGIA, USA, pages 649 - 659, XP010584603**

**Description**

## BACKGROUND

The field of biotechnology has grown tremendously in the last 20 years. This growth has been due to many factors, some of which include the improvements in the equipment available for bioreactors, the increased understanding of biological systems and increased knowledge as to the interactions of materials (such as monoclonal antibodies and recombinant proteins) with the various systems of the human body.

Improvements in equipment have allowed for larger volumes and lower cost for the production of biologically derived materials such as recombinant proteins. This is especially prevalent in the area of pharmaceuticals, where the successes of many types of new drug therapies have been directly due to the ability to mass produce these materials through protein-based manufacturing methods.

One of the key components that is utilized in the manufacturing processes of new biologically based pharmaceuticals is the bioreactor and the ancillary processes associated therewith. An area of growth in the bioreactor field has been with the perfusion process. The perfusion process is distinguished from the fed-batch process by its lower capital cost and higher throughput.

In the fed-batch process, a culture is seeded in a bioreactor. The gradual addition of a fresh volume of selected nutrients during the growth cycle is used to improve productivity and growth. The product is recovered after the culture is harvested. The fed batch bioreactor process has been attractive because of its simplicity and also due to carryover from well-known fermentation processes. However, a fed-batch bioreactor has high start-up costs, and generally has a large volume to obtain a cost-effective amount of product at the end of the growth cycle. After the batch is completed, the bioreactor must be cleaned and sterilized, resulting in nonproductive downtime.

A perfusion bioreactor processes a continuous supply of fresh media that is fed into the bioreactor while growth-inhibiting byproducts are constantly removed. The nonproductive downtime can be reduced or eliminated with a perfusion bioreactor process. The cell densities achieved in perfusion culture (30-100 million cells/mL) are typically higher than for fed-batch modes (5-25 million cells/mL). However, a perfusion bioreactor requires a cell retention device to prevent escape of the culture when byproducts are being removed. These cell retention systems add a level of complexity to the perfusion process, requiring management, control, and maintenance for successful operation. Operational issues such as malfunction or failure of the cell retention equipment has previously been a problem with perfusion bioreactors. This has limited their attractiveness in the past

Document US2011/123392 A1 and "Ultrasonic separation of suspended particles" (2001 IEEE ULTRASONICS SYMPOSIUM PROCEEDINGS, ATLANTA, GA, OCT. 7 - 10, 2001; pages 649-659) describe ultrasonically enhanced separation devices for bioreactors.

## BRIEF DESCRIPTION

The present disclosure relates, in various embodiments, to a system for producing biomolecules such as recombinant proteins or monoclonal antibodies, and for separating these desirable products from a cell culture in a bioreactor. Generally, a fluid medium containing the cells and the desired products are passed or flowed through a filtering device. The system us set up for use as a perfusion reactor.

Disclosed in various embodiments is a system according to claim 1 comprising a bioreactor and a filtering device. The bioreactor includes a reaction vessel, an agitator, a feed inlet, and an outlet. The filtering device comprises: an inlet fluidly connected to the bioreactor outlet for receiving fluid from the bioreactor; a flow chamber through which the fluid can flow; and a sleeve surrounding the flow chamber, the sleeve including at least one ultrasonic transducer and a reflector located opposite the at least one ultrasonic transducer, the at least one ultrasonic transducer being driven to produce a multi-dimensional standing wave in the flow chamber.

The filtering device further comprises a product outlet through which desired product is recovered. The filtering device further comprises a recycle outlet for sending fluid back to the bioreactor.

The multi-dimensional standing wave has an axial force component and a lateral force component which are of the same order of magnitude. The bioreactor can be operated as a perfusion bioreactor.

The sleeve may be separable from the flow chamber. Sometimes, the filtering device further comprises a jacket located between the sleeve and the flow chamber, the jacket being used to regulate the temperature of the fluid in the flow chamber. The jacket, the sleeve, and the flow chamber can be separable from each other and be disposable.

In particular embodiments, the ultrasonic transducer comprises a piezoelectric material that can vibrate in a higher order mode shape. The piezoelectric material may have a rectangular shape.

The ultrasonic transducer may comprise: a housing having a top end, a bottom end, and an interior volume; and a crystal at the bottom end of the housing having an exposed exterior surface and an interior surface, the crystal being able to vibrate when driven by a voltage signal. In some embodiments, a backing layer contacts the interior surface of the crystal, the backing layer being made of a substantially acoustically transparent material. The substantially acoustically transparent material can be balsa wood, cork, or foam. The substantially acoustically transparent material may have a thickness of up to 25,4 mm (1 inch and 1" = 25,4 mm applies to all documents). The substantially acoustically transparent material can be in the form of a lattice. In other embodiments, an exterior surface of the crystal is covered by a wear surface material with a thickness of a half wavelength or less, the wear surface material being a urethane, epoxy, or silicone coating. In yet other embodiments, the crystal has no backing layer or wear layer.

The multi-dimensional standing wave is a three-dimensional standing wave.

The reflector may have a non-planar surface.

These and other characteristics are more particularly described below. The present disclosure in particular relates to a system according to present claim 1 and a method according to present claim 13.

## BRIEF DESCRIPTION OF THE DRAWINGS

The following is a brief description of the drawings, which are presented for the purposes of illustrating the exemplary embodiments disclosed herein and not for the purposes of limiting the same.

**Figure 1** illustrates a single standing acoustic wave generated by an ultrasonic transducer and a reflector.

**Figure 2** is an illustration comparing a fed-batch bioreactor system without an acoustophoretic filtering device with a perfusion bioreactor system with an acoustophoretic filtering device.

**Figure 3** is a cross-sectional view that shows the various components of a bioreactor.

**Figure 4** shows one embodiment of an acoustophoretic filtering device of the present disclosure, with a sleeve surrounding a pipe that acts as a flow chamber and is disposable.

**Figure 5** shows another embodiment of an acoustophoretic filtering device of the present disclosure, showing a jacket surrounding the flow chamber, and the sleeve surrounding the jacket. The sleeve contains a fluid that is used to regulate the temperature of the fluid passing through the flow chamber.

**Figure 6** is a schematic view illustrating a system of the present disclosure, including a perfusion bioreactor with an acoustophoretic separation device, and a recycle path.

**Figure 7** is a cross-sectional diagram of a conventional ultrasonic transducer.

**Figure 8** is a picture of a wear plate of a conventional transducer.

**Figure 9** is a cross-sectional diagram of an ultrasonic transducer of the present disclosure. An air gap is present within the transducer, and no backing layer or wear plate is present.

**Figure 10** is a cross-sectional diagram of an ultrasonic transducer of the present disclosure. An air gap is present within the transducer, and a backing layer and wear plate are present.

**Figure 11** is a graph of electrical impedance amplitude versus frequency for a square transducer driven at different frequencies.

**Figure 12** illustrates the trapping line configurations for seven of the peak amplitudes of **Figure 11** from the direction orthogonal to fluid flow.

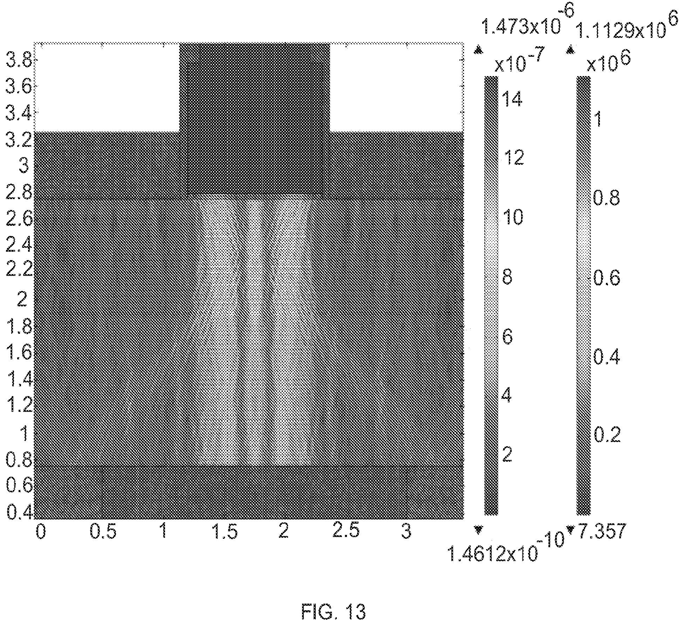

**Figure 13** is a computer simulation of the acoustic pressure amplitude (right-hand scale in Pa) and transducer out of plane displacement (left-hand scale in meters). The text at the top of the left-hand scale reads "x10$^{-7}$". The text at the top of the left-hand scale by the upward-pointing triangle reads "1.473x10$^{-6}$". The text at the bottom of the left-hand scale by the downward-pointing triangle reads "1.4612x10$^{-10}$". The text at the top of the right-hand scale reads "x10$^{6}$". The text at the top of the right-hand scale by the upward-pointing triangle reads "1.1129x10$^{6}$". The text at the bottom of the right-hand scale by the downward-pointing triangle reads "7.357". The triangles show the maximum and minimum values depicted in this figure for the given scale. The horizontal axis is the location within the chamber along the X-axis, in inches, and the vertical axis is the location within the chamber along the Y-axis, in inches.

**Figure 14** shows the In-Plane and Out-of-Plane displacement of a crystal where composite waves are present.

**Figure 15** shows an exploded view of an acoustophoretic separator used for conducting some example separations, having one flow chamber.

**Figure 16** shows an exploded view of a stacked acoustophoretic separator with two flow chambers.

**Figure 17** is a graph showing the efficiency of removing cells from a medium using a Beckman Coulter Cell Viability Analyzer for one experiment.

**Figure 18** is a graph showing the efficiency of removing cells from a medium using a Beckman Coulter Cell Viability Analyzer for another experiment.

## DETAILED DESCRIPTION

The present disclosure may be understood more readily by reference to the following detailed description of desired embodiments and the examples included therein. In the following specification and the claims which follow, reference will be made to a number of terms which shall be defined to have the following meanings.

It should be noted that many of the terms used herein are relative terms. For example, the terms "upper" and "lower" are relative to each other in location, i.e. an upper component is located at a higher elevation than a lower component in a given orientation, but these terms can change if the device is flipped. The terms "inlet" and "outlet" are relative to a fluid flowing through them with respect to a given structure, e.g. a fluid flows through the inlet into the structure and flows through the outlet out of the structure. The terms "upstream" and "downstream" are relative to the direction in which a fluid flows through various components, i.e. the flow fluids through an upstream component prior to flowing through the downstream component. It should be noted that in a loop, a first component can be described as being both upstream of and downstream of a second component.

The present application refers to "the same order of magnitude." Two numbers are of the same order of magnitude if the quotient of the larger number divided by the smaller number is a value less than 10.

Bioreactors are useful for making biomolecules such as recombinant proteins or monoclonal antibodies. Very generally, cells are cultured in a bioreactor vessel with media in order to produce the desired product, and the desired product is then harvested by separation from the cells and media. The use of mammalian cell cultures including Chinese hamster ovary (CHO), NS0 hybridoma cells, baby hamster kidney (BHK) cells, and human cells has proven to be a very efficacious way of producing/expressing the recombinant proteins and monoclonal antibodies required of today's pharmaceuticals. Two general types of bioreactor processes exist: fed-batch and perfusion.

While fed-batch reactors are the norm currently, due mainly to the familiarity of the process to many scientists and technicians, perfusion technology is growing at a very fast clip. Many factors favor the use of a perfusion bioreactor process. The capital and start-up costs for perfusion bioreactors are lower, smaller upstream and downstream capacity is required, and the process uses smaller volumes and fewer seed steps than fed-batch methods. A perfusion bioreactor process also lends itself better to development, scale-up, optimization, parameter sensitivity studies, and validation.

Recent developments in perfusion bioreactor technology also favor its use. Control technology and general support equipment is improving for perfusion bioreactors, increasing the robustness of perfusion processes. The perfusion process can now be scaled up to bioreactors having a volume up to 1000 liters (L). Better cell retention systems for perfusion bioreactors result in lower cell loss and greater cell densities than have been seen previously. Cell densities greater than 50 million cells/mL are now achievable, compared to fed-batch cell densities of around 20 million cells/mL. Lower contamination and infection rates have improved the output of perfusion bioreactors. Higher product concentrations in the harvest and better yields without significant increase in cost have thus resulted for perfusion processes.

A separate aspect of the use of high cell concentration bioreactors is the "dewatering" of the materials at the end of a bioreactor run. The "dewatering" or removal of interstitial fluid from a bioreactor sludge is important for improving the efficiency of recovery of the intended bioreactor product. Currently, high energy centrifuges with internal structures (known as disk stack centrifuges) are utilized to remove the interstitial fluid from the bioreactor sludge at the end of a run. The capital cost and operating costs for a disk stack centrifuge is high. A simpler method of removing the interstitial fluid from the remaining bioreactor sludge that can be performed without the high capital and operating costs associated with disk stack centrifuges is desirable. In addition, current methods of filtration or centrifugation can damage cells, releasing protein debris and enzymes into the purification process and increasing the load on downstream portions of the purification system.

Briefly, the present disclosure relates to the generation of three-dimensional (3-D) acoustic standing waves from one or more piezoelectric transducers, where the transducers are electrically or mechanically excited such that they move in a "drumhead" or multi-excitation mode rather than a "piston" or single excitation mode fashion. Through this manner of wave generation, a higher lateral trapping force is generated than if the piezoelectric transducer is excited in a "piston" mode where only one large standing wave is generated. Thus, with the same input power to a piezoelectric transducer, the 3-D acoustic standing waves can have a higher lateral trapping force compared to a single acoustic standing wave. This can be used to facilitate proteinaceous fluid purification of the contents of a bioreactor. Thus, the present disclosure relates to processing systems comprising a bioreactor and a filtering device, the filtering device using acoustophoresis for separation of various components.

Through utilization of an acoustophoretic filtering device that incorporates a 3-D standing wave, maintaining flux rates and minimizing cross-contamination risk in a multiproduct system can also be achieved. Other benefits, such as cleaning procedures and related demands often detailed and validated within standard operating procedures (SOP), can also be realized through the use of a 3-D acoustic standing wave capable apparatus. The cross-contamination risk can be eliminated between the bioreactor and outside processes.

Acoustophoresis is a low-power, no-pressure-drop, no-clog, solid-state approach to particle removal from fluid dispersions: i.e., it is used to achieve separations that are more typically performed with porous filters, but it has none of the disadvantages of filters. In particular, the present disclosure provides filtering devices that are suitable for use with bioreactors and operate at the macro-scale for separations in flowing systems with high flow rates. The acoustophoretic filtering device is designed to create a high intensity three dimensional ultrasonic standing wave that results in an acoustic radiation force that is larger than the combined effects of fluid drag and buoyancy or gravity, and is therefore able to trap (i.e., hold stationary) the suspended phase (i.e. cells) to allow more time for the acoustic wave to increase particle concentration, agglomeration and/or coalescence. The present systems have the ability to create ultrasonic standing wave fields that can trap particles in flow fields with a linear velocity ranging from 0.1 mm/sec to velocities exceeding 1 cm/s. This technology offers a green and sustainable alternative for separation of secondary phases with a significant reduction in cost of energy. Excellent particle separation efficiencies have been demonstrated for particle sizes as small as one micron.

The ultrasonic standing waves can be used to trap, i.e., hold stationary, secondary phase particles (e.g. cells) in a host fluid stream (e.g. cell culture media). This is an important distinction from previous approaches where particle trajectories were merely altered by the effect of the acoustic radiation force. The scattering of the acoustic field off the particles results in a three dimensional acoustic radiation force, which acts as a three-dimensional trapping field. The acoustic radiation force is proportional to the particle volume (e.g. the cube of the radius) when the particle is small relative to the wavelength. It is proportional to frequency and the acoustic contrast factor. It also scales with acoustic energy (e.g. the square of the acoustic pressure amplitude). For harmonic excitation, the sinusoidal spatial variation of the force is what drives the particles to the stable positions within the standing waves. When the acoustic radiation force exerted on the particles is stronger than the combined effect of fluid drag force and buoyancy/gravitational force, the particle is trapped within the acoustic standing wave field. The action of the acoustic forces on the trapped particles results in concentration, agglomeration and/or coalescence of particles. Additionally, secondary inter-particle forces, such as Bjerkness forces, aid in particle agglomeration.

Generally, the 3-D standing wave(s) filtering system is operated at a voltage such that the protein-producing materials, such as Chinese hamster ovary cells (CHO cells), the most common host for the industrial production of recombinant protein therapeutics, are trapped in the ultrasonic standing wave, i.e., remain in a stationary position. Within each nodal plane, the CHO cells are trapped in the minima of the acoustic radiation potential. Most cell types present a higher density and lower compressibility than the medium in which they are suspended, so that the acoustic contrast factor between the cells and the medium has a positive value. As a result, the axial acoustic radiation force (ARF) drives the CHO cells towards the standing wave pressure nodes. The axial component of the acoustic radiation force drives the cells, with a positive contrast factor, to the pressure nodal planes, whereas cells or other particles with a negative contrast factor are driven to the pressure anti-nodal planes. The radial or lateral component of the acoustic radiation force is the force that traps the cells. The radial or lateral component of the ARF is larger than the combined effect of fluid drag force and gravitational force. For small cells or emulsions the drag force $F_D$ can be expressed as:

$$\vec{F}_D = 4\pi\mu_f R_p \left(\vec{U}_f - \vec{U}_p\right) \left[\frac{1 + \frac{3}{2}\hat{\mu}}{1 + \hat{\mu}}\right],$$

where $U_f$ and $U_p$ are the fluid and cell velocity, $R_p$ is the particle radius, $\mu_f$ and $\mu_p$ are the dynamic viscosity of the fluid and the cells, and $\hat{\mu} = \mu_p/\mu_f$ is the ratio of dynamic viscosities. The buoyancy force $F_B$ is expressed as:

$$F_B = \frac{4}{3}\pi R_p^3 \left(\rho_f - \rho_p\right).$$

For a cell to be trapped in the ultrasonic standing wave, the force balance on the cell must be zero, and therefore an expression for lateral acoustic radiation force $F_{LRF}$ can be found, which is given by:

$$F_{LRF} = F_D + F_B.$$

For a cell of known size and material property, and for a given flow rate, this equation can be used to estimate the magnitude of the lateral acoustic radiation force.

The theoretical model that is used to calculate the acoustic radiation force is based on the formulation developed by Gor'kov.[17] The primary acoustic radiation force $F_A$ is defined as a function of a field potential U, $F_A = -\nabla(U)$, where the field potential U is defined as

$$U = V_0 \left[ \frac{\langle p^2 \rangle}{2\rho_f c_f^{\ 2}} f_1 - \frac{3\rho_f \langle u^2 \rangle}{4} f_2 \right],$$

and $f_1$ and $f_2$ are the monopole and dipole contributions defined by

$$f_1 = 1 - \frac{1}{\Lambda \sigma^2},$$

$$f_2 = \frac{2(\Lambda - 1)}{2\Lambda + 1},$$

where $p$ is the acoustic pressure, $u$ is the fluid particle velocity, $\Lambda$ is the ratio of cell density $\rho_p$ to fluid density $\rho_f$, $\sigma$ is the ratio of cell sound speed $c_p$ to fluid sound speed $c_f$, $V_o$ is the volume of the cell, and < > indicates time averaging over the period of the wave.

The lateral force of the total acoustic radiation force (ARF) generated by the ultrasonic transducers of the present disclosure is significant and is sufficient to overcome the fluid drag force at linear velocities of up to 1 cm/s. This lateral ARF can thus be used to retain cells in a bioreactor while the bioreactor process continues. This is especially true for a perfusion bioreactor.

The filtering devices of the present disclosure, which use ultrasonic transducers and acoustophoresis, can also improve the dewatering of the leftover material from a bioreactor batch (i.e bioreactor sludge), and thus reduce the use of or eliminate the use of disk stack centrifuges. This simplifies processing and reduces costs.

In a perfusion bioreactor system, it is desirable to be able to filter and separate the cells and cell debris from the expressed materials that are in the fluid stream (i.e. cell culture media). The expressed materials are composed of biomolecules such as recombinant proteins or monoclonal antibodies, and are the desired product to be recovered.

An acoustophoretic filtering device can be used in at least two different ways. First, the standing waves can be used to trap the expressed biomolecules and separate this desired product from the cells, cell debris, and media. The expressed biomolecules can then be diverted and collected for further processing. Alternatively, the standing waves can be to trap the cells and cell debris present in the cell culture media. The cells and cell debris, having a positive contrast factor, move to the nodes (as opposed to the anti-nodes) of the standing wave. As the cells and cell debris agglomerate at the nodes of the standing wave, there is also a physical scrubbing of the cell culture media that occurs whereby more cells are trapped as they come in contact with the cells that are already held within the standing wave. This generally separates the cells and cellular debris from the cell culture media. When the cells in the standing wave agglomerate to the extent where the mass is no longer able to be held by the acoustic wave, the aggregated cells and cellular debris that have been trapped can fall out of the fluid stream through gravity, and can be collected separately. To aid this gravitational settling of the cells and cell debris, the standing wave may be interrupted to allow all of the cells to fall out of the fluid stream that is being filtered. This process can be useful for dewatering. The expressed biomolecules may have been removed beforehand, or remain in the fluid stream (i.e. cell culture medium).

Desirably, the ultrasonic transducer(s) generate a three-dimensional standing wave in the fluid that exerts a lateral force on the suspended particles to accompany the axial force so as to increase the particle trapping capabilities of the acoustophoretic filtering device. Typical results published in literature state that the lateral force is two orders of magnitude smaller than the axial force. In contrast, the technology disclosed in this application provides for a lateral force to be of the same order of magnitude as the axial force.

The acoustic filtering devices of the present disclosure are designed to maintain a high intensity three-dimensional acoustic standing wave. The device is driven by a function generator and amplifier (not shown). The device performance is monitored and controlled by a computer. It may be necessary, at times, due to acoustic streaming, to modulate the frequency or voltage amplitude of the standing wave. This may be done by amplitude modulation and/or by frequency modulation. The duty cycle of the propagation of the standing wave may also be utilized to achieve certain results for trapping of materials. In other words, the acoustic beam may be turned on and shut off at different frequencies to achieve desired results.

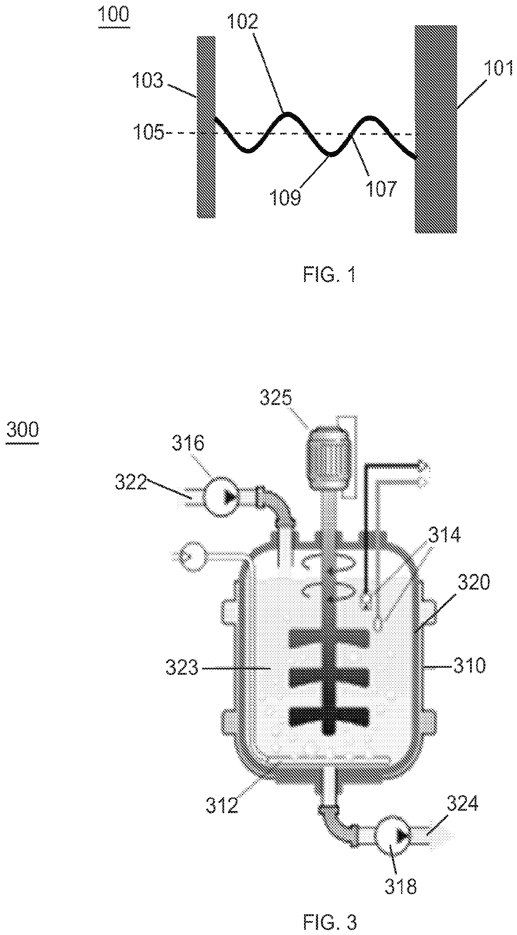

**Figure 1** illustrates a single standing wave system **100** that is comprised of a reflector plate **101** and an ultrasonic transducer **103** that is set to resonate so as to form a standing wave **102**. Excitation frequencies typically in the range from hundreds of kHz to tens of MHz are applied by the transducer **103**. One or more standing waves are created between the transducer **103** and the reflector **101**. The standing wave is the sum of two propagating waves that are equal in frequency and intensity and that are traveling in opposite directions, i.e. from the transducer to the reflector and back. The propagating waves destructively interfere with each other and thus generate the standing wave. A dotted line **105** is used to indicate the amplitude. A node is a point where the wave has minimum amplitude, and is indicated with reference numeral **107.** An anti-node is a point where the wave has maximum amplitude, and is indicated with reference numeral **109.**

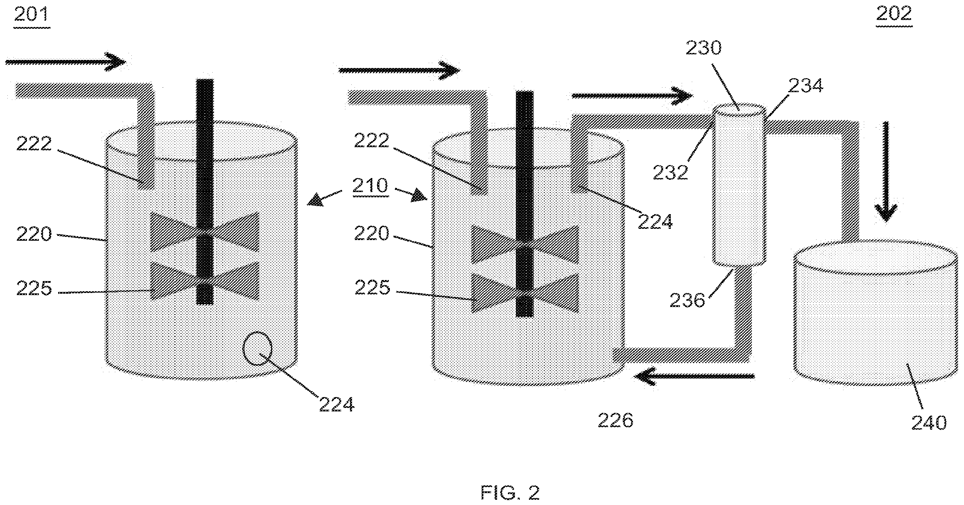

**Figure 2** is a schematic diagram that compares a fed-batch bioreactor system **201** without an acoustophoretic filtering device (left side, not an embodiment of the invention) with a perfusion bioreactor system **202** with an acoustophoretic filtering device (right side, an embodiment of the invention). Beginning with the fed-batch bioreactor on the left, the bioreactor **210** includes a reaction vessel **220**. The cell culture media is fed to the reaction vessel through a feed inlet **222.** An agitator **225** is used to circulate the media throughout the cell culture. Here, the agitator is depicted as a set of rotating blades, though any type of system that causes circulation is contemplated. The bioreactor permits growth of a seed culture through a growth / production cycle, during which time debris, waste and unusable cells will accumulate in the bioreactor and the desired product (e.g. biomolecules such as monoclonal antibodies, recombinant proteins, hormones, etc.) will be produced as well. Due to this accumulation, the reaction vessel of a fed-batch process is typically much larger than that in a perfusion process. The desired product is then harvested at the end of the production cycle. The reaction vessel **220** also includes an outlet **224** for removing material.

Turning now to the perfusion bioreactor **202** on the right-hand side, again, the bioreactor includes a reaction vessel **220** with a feed inlet **222** for the cell culture media. An agitator **225** is used to circulate the media throughout the cell culture. An outlet **224** of the reaction vessel is fluidly connected to the inlet **232** of a filtering device **230**, and continuously feeds the media (containing cells and desired product) to the filtering device. The filtering device is located downstream of the reaction vessel, and separates the desired product from the cells. The filtering device **230** has two separate outlets, a product outlet **234** and a recycle outlet **236**. The product outlet **234** fluidly connects the filtering device **230** to a containment vessel **240** downstream of the filtering device, which receives a concentrated flow of the desired product (plus media) from the filtering device. From there, further processing / purification can occur to isolate / recover the desired product. The recycle outlet **236** fluidly connects the filtering device **230** back to a recycle inlet **226** of the reaction vessel **220,** and is used to send the cells and cell culture media back into the reaction vessel for continued growth / production. Put another way, there is a fluid loop between the reaction vessel and the filtering device. The reaction vessel **220** in the perfusion bioreactor system **202** has a continuous throughput of product and thus can be made smaller. The filtering process is critical to the throughput of the perfusion bioreactor. A poor filtering process will allow for only low throughput and result in low yields of the desired product.

**Figure 3** is a cross-sectional view of a generic bioreactor **300** that is useful for the systems of the present disclosure. As illustrated here, the bioreactor includes a reaction vessel **320** having an internal volume **323**. A feed inlet **322** at the top of the vessel is used to feed cell culture media into the vessel. An agitator **325** is present. An outlet **324** is shown at the bottom of the vessel. A thermal jacket **310** surrounds the reaction vessel, and is used to regulate the temperature of the cells / media. An aerator **312** is located on the bottom of the vessel for providing gas to the internal volume. Sensors **314** are shown at the top right of the vessel. A pump **316** is illustrated for feeding the cell culture media into the vessel, as is another pump **318** for removing cell culture media from the vessel.

The perfusion systems of the present disclosure also use an acoustophoretic filtering device. The contents of the bioreactor are continuously flowed through the filtering device to capture the desired products.

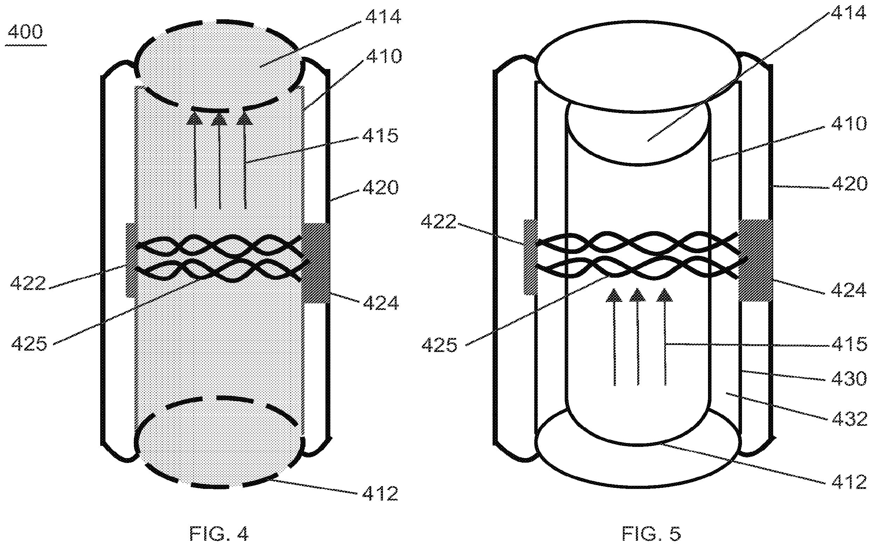

**Figure 4** is a first embodiment of an acoustophoretic filtering device **400**. The device includes a flow chamber **410,** which is depicted here as a cylindrical pipe or tube. A feed inlet **412** is illustrated here at the bottom of the flow chamber, through which fluid from the bioreactor is received. An outlet **414** is depicted at the top of the flow chamber, with the arrows (reference numeral **415**) indicating the direction of fluid flow. A sleeve **420** surrounds the flow chamber. The sleeve includes at least one ultrasonic transducer **422** and at least one reflector **424,** which are located opposite each other. Together, the transducer and reflector generate one or more standing waves **425**, with the reflector bouncing the initial propagated wave back towards the transducer with a similar frequency and intensity to form an acoustic standing wave. It is particularly contemplated that the sleeve can be separated from the flow chamber / pipe. The pipe can be discarded and replaced with a new pipe. This allows for disposable parts in the filtering device, and thus reduces the cost of cleaning and sterilization that might otherwise be incurred with a permanent filter. It is noted that the filtering device may include additional inlets or outlets not depicted here, as previously explained.

**Figure 5** is a second embodiment of the acoustophoretic filtering device. Here, the filtering device **400** also includes a jacket **430** that is located between the sleeve **420** and the flow chamber **410.** The jacket contains a temperature-regulating fluid **432** that can be used to control the temperature of the fluid passing through the flow chamber. In this regard, it is usually desirable to maintain the temperature of the cell culture below 38°C to prevent compromise of the cells. The temperature-regulating fluid is completely separated from the cell culture media/fluid passing through the flow chamber **410.** It is noted that the standing wave **425** created by the transducer **422** and reflector **424** will propagate through the jacket **430** and the temperature regulating fluid **432** therein, and will continue to operate in the flow chamber to separate the targeted material in the flow chamber.

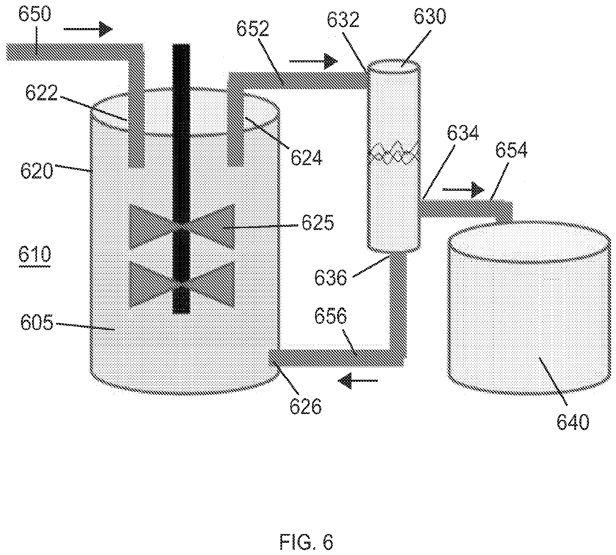

**Figure 6** illustrates an exemplary processing system of the present disclosure, comprising a bioreactor **610** and a filtering device **630**. The system is set up for use as a perfusion bioreactor. The bioreactor **610** includes a reaction vessel **620** having a feed inlet **622**, an outlet **624**, and a recycle inlet **626**. Media is added into the feed inlet **622** by an addition pipe **650**. The contents of the reaction vessel (reference numeral **605**) are mixed with an agitator **625**. The desired product (e.g. recombinant proteins) is continuously produced by cells located within the vessel **620**, and are present in the media of the bioreactor. The product and the cells in the perfusion bioreactor are drawn from the reaction vessel through pipe **652**, and enter the acoustophoretic filtering device **630** through inlet **632**. There, the desired product is separated from the cells through the use of multi-dimensional standing waves. The desired product can be drawn off through a product outlet **634** and pipe **654** into a containment vessel **640**. The cells are returned to the perfusion bioreactor after separation, passing from recycle outlet **636** of the filtering device through pipe **656** to recycle inlet **626** of the reaction vessel, which form a recycle path. The 3-D standing waves of the acoustophoresis device allow for high throughput of the perfusion reactor due to the increased lateral trapping force of the 3-D standing waves. It is noted that although the reaction vessel outlet **624** is depicted at the top of the vessel and the recycle inlet **626** is depicted at the bottom of the vessel, that this arrangement can be reversed if desired. This may depend on the desired product to be obtained.

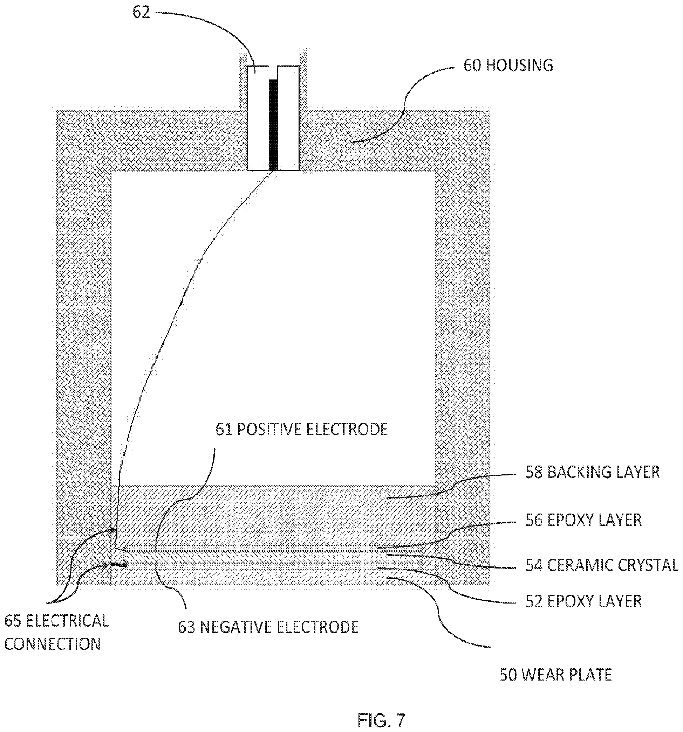

It may be helpful now to describe the ultrasonic transducer(s) used in the acoustophoretic filtering device in more detail. **Figure 7** is a cross-sectional diagram of a conventional ultrasonic transducer. This transducer has a wear plate **50** at a bottom end, epoxy layer **52**, ceramic crystal **54** (made of, e.g. Lead Zirconate Titanate (PZT)), an epoxy layer **56**, and a backing layer **58**. On either side of the ceramic crystal, there is an electrode: a positive electrode **61** and a negative electrode **63**. The epoxy layer **56** attaches backing layer **58** to the crystal **54**. The entire assembly is contained in a housing **60** which may be made out of, for example, aluminum. An electrical adapter **62** provides connection for wires to pass through the housing and connect to leads (not shown) which attach to the crystal **54**. Typically, backing layers are designed to add damping and to create a broadband transducer with uniform displacement across a wide range of frequency and are designed to suppress excitation at particular vibrational eigen-modes. Wear plates are usually designed as impedance transformers to better match the characteristic impedance of the medium into which the transducer radiates.

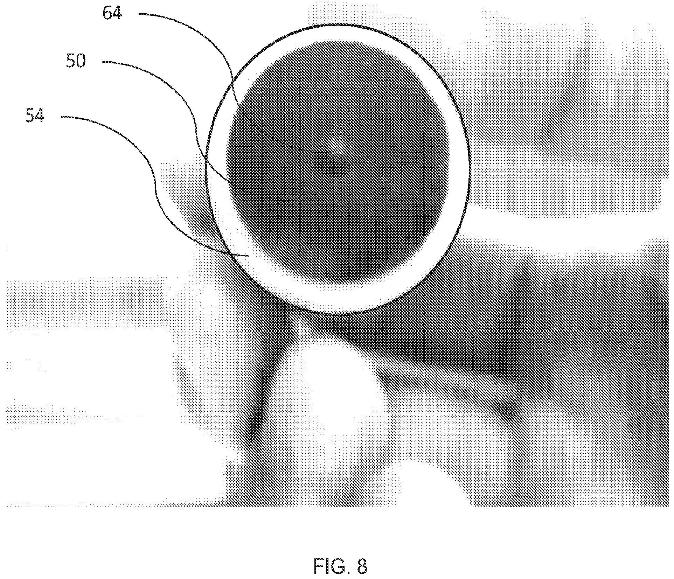

**Figure 8** is a photo of a wear plate **50** with a bubble **64** where the wear plate has pulled away from the ceramic crystal surface due to the oscillating pressure and heating.

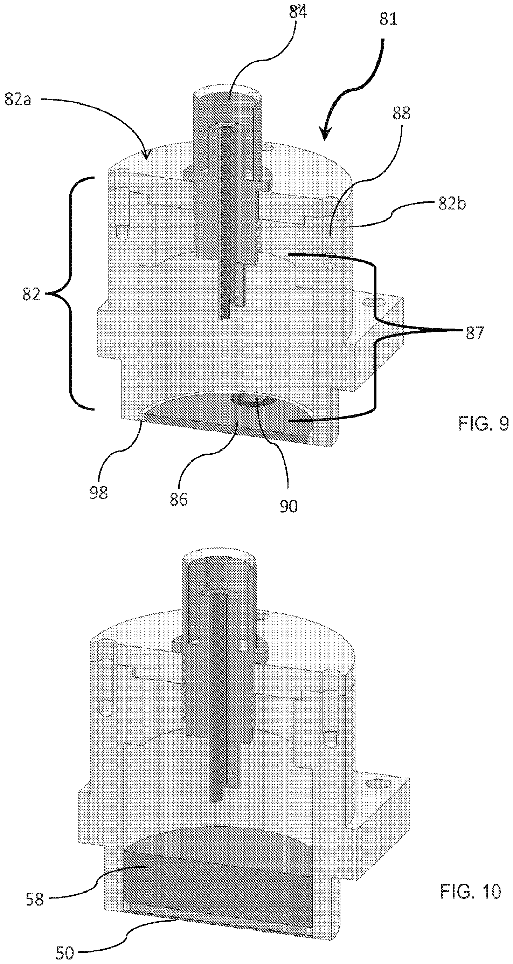

**Figure 9** is a cross-sectional view of an ultrasonic transducer **81** of the present disclosure, which is used in the acoustophoretic filtering devices of the present disclosure. Transducer **81** has an aluminum housing **82**. A PZT crystal **86** defines the bottom end of the transducer, and is exposed from the exterior of the housing. The crystal is supported on its perimeter by a small elastic layer **98**, e.g. silicone or similar material, located between the crystal and the housing. Put another way, no wear layer is present.

Screws (not shown) attach an aluminum top plate **82a** of the housing to the body **82b** of the housing via threads **88**. The top plate includes a connector **84** to pass power to the PZT crystal **86**. The bottom and top surfaces of the PZT crystal **86** are each connected to an electrode (positive and negative), such as silver or nickel. A wrap-around electrode tab **90** connects to the bottom electrode and is isolated from the top electrode. Electrical power is provided to the PZT crystal **86** through the electrodes on the crystal, with the wrap-around tab **90** being the ground connection point. Note that the crystal **86** has no backing layer or epoxy layer as is present in **Figure 5**. Put another way, there is an air gap **87** in the transducer between aluminum top plate **82a** and the crystal **86** (i.e. the air gap is completely empty). A minimal backing **58** and/or wear plate **50** may be provided in some embodiments, as seen in **Figure 10.**

The transducer design can affect performance of the system. A typical transducer is a layered structure with the ceramic crystal bonded to a backing layer and a wear plate. Because the transducer is loaded with the high mechanical impedance presented by the standing wave, the traditional design guidelines for wear plates, e.g., half wavelength thickness for standing wave applications or quarter wavelength thickness for radiation applications, and manufacturing methods may not be appropriate. Rather, in one embodiment of the present disclosure the transducers, there is no wear plate or backing, allowing the crystal to vibrate in one of its eigenmodes with a high Q-factor. The vibrating ceramic crystal/disk is directly exposed to the fluid flowing through the flow chamber.

Removing the backing (e.g. making the crystal air backed) also permits the ceramic crystal to vibrate at higher order modes of vibration with little damping (e.g. higher order modal displacement). In a transducer having a crystal with a backing, the crystal vibrates with a more uniform displacement, like a piston. Removing the backing allows the crystal to vibrate in a non-uniform displacement mode. The higher order the mode shape of the crystal, the more nodal lines the crystal has. The higher order modal displacement of the crystal creates more trapping lines, although the correlation of trapping line to node is not necessarily one to one, and driving the crystal at a higher frequency will not necessarily produce more trapping lines.

In some embodiments, the crystal may have a backing that minimally affects the Q-factor of the crystal (e.g. less than 5%). The backing may be made of a substantially acoustically transparent material such as balsa wood, foam, or cork which allows the crystal to vibrate in a higher order mode shape and maintains a high Q-factor while still providing some mechanical support for the crystal. The backing layer may be a solid, or may be a lattice having holes through the layer, such that the lattice follows the nodes of the vibrating crystal in a particular higher order vibration mode, providing support at node locations while allowing the rest of the crystal to vibrate freely. The goal of the lattice work or acoustically transparent material is to provide support without lowering the Q-factor of the crystal or interfering with the excitation of a particular mode shape.

Placing the crystal in direct contact with the fluid also contributes to the high Q-factor by avoiding the dampening and energy absorption effects of the epoxy layer and the wear plate. Other embodiments may have wear plates or a wear surface to prevent the PZT, which contains lead, contacting the host fluid. This may be desirable in, for example, biological applications such as separating blood. Such applications might use a wear layer such as chrome, electrolytic nickel, or electroless nickel. Chemical vapor deposition could also be used to apply a layer of poly(p-xylylene) (e.g. Parylene) or other polymer. Organic and biocompatible coatings such as silicone or polyurethane are also usable as a wear surface.

In some embodiments, the ultrasonic transducer has a 1 inch diameter and a nominal 2 MHz resonance frequency. Each transducer can consume about 28 W of power for droplet trapping at a flow rate of 11,3562 litres per minute (3 GPM). This translates to an energy cost of 0.25 kW hr/m$^3$. This is an indication of the very low cost of energy of this technology. Desirably, each transducer is powered and controlled by its own amplifier. In other embodiments, the ultrasonic transducer uses a square crystal, for example with 1"x1" dimensions. Alternatively, the ultrasonic transducer can use a rectangular crystal, for example with 1"x2.5" dimensions. Power dissipation per transducer was 10 W per 1"x1" transducer cross-sectional area and per inch of acoustic standing wave span in order to get sufficient acoustic trapping forces. For a 4" span of an intermediate scale system, each 1"x1" square transducer consumes 40 W. The larger 1"x2.5" rectangular transducer uses 100W in an intermediate scale system. The array of three 1"x1" square transducers would consume a total of 120 W and the array of two 1"x2.5" transducers would consume about 200 W. Arrays of closely spaced transducers represent alternate potential embodiments of the technology. Transducer size, shape, number, and location can be varied as desired to generate desired three-dimensional acoustic standing wave patterns.

The size, shape, and thickness of the transducer determine the transducer displacement at different frequencies of excitation, which in turn affects separation efficiency. Typically, the transducer is operated at frequencies near the thickness resonance frequency (half wavelength). Gradients in transducer displacement typically result in more trapping locations for the cells/biomolecules. Higher order modal displacements generate three-dimensional acoustic standing waves with strong gradients in the acoustic field in all directions, thereby creating equally strong acoustic radiation forces in all directions, leading to multiple trapping lines, where the number of trapping lines correlate with the particular mode shape of the transducer.

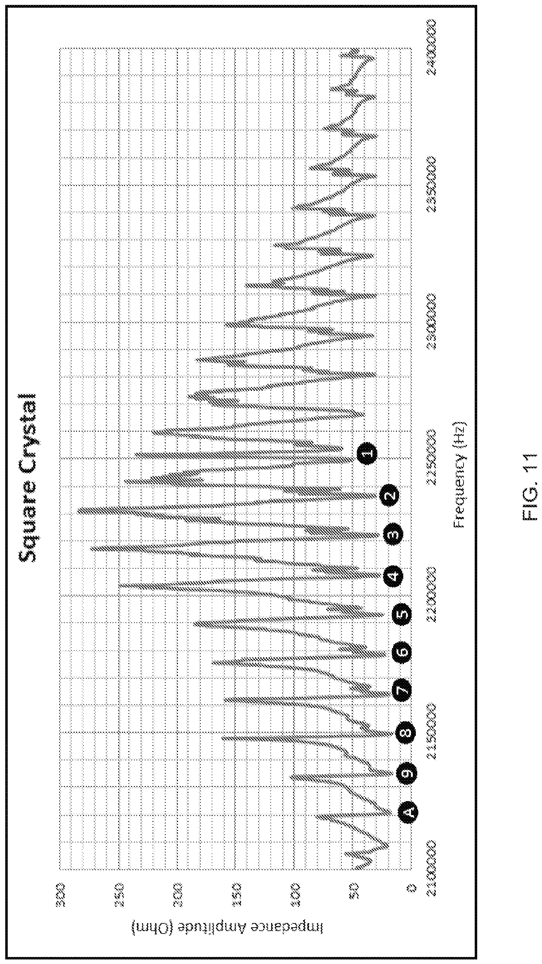

To investigate the effect of the transducer displacement profile on acoustic trapping force and separation efficiencies, an experiment was repeated ten times using a 1"x1" square transducer, with all conditions identical except for the excitation frequency. Ten consecutive acoustic resonance frequencies, indicated by circled numbers 1-9 and letter A on **Figure 11**, were used as excitation frequencies. The conditions were experiment duration of 30 min, a 1000 ppm oil concentration of approximately 5-micron SAE-30 oil droplets, a flow rate of 500 ml/min, and an applied power of 20W. Oil droplets were used because oil is denser than water, and can be separated from water using acoustophoresis.

**Figure 11** shows the measured electrical impedance amplitude of a square transducer as a function of frequency in the vicinity of the 2.2 MHz transducer resonance. The minima in the transducer electrical impedance correspond to acoustic resonances of the water column and represent potential frequencies for operation. Numerical modeling has indicated that the transducer displacement profile varies significantly at these acoustic resonance frequencies, and thereby directly affects the acoustic standing wave and resulting trapping force. Since the transducer operates near its thickness resonance, the displacements of the electrode surfaces are essentially out of phase. The typical displacement of the transducer electrodes is not uniform and varies depending on frequency of excitation. As an example, at one frequency of excitation with a single line of trapped oil droplets, the displacement has a single maximum in the middle of the electrode and minima near the transducer edges. At another excitation frequency, the transducer profile has multiple maxima leading to multiple trapped lines of oil droplets. Higher order transducer displacement patterns result in higher trapping forces and multiple stable trapping lines for the captured oil droplets.

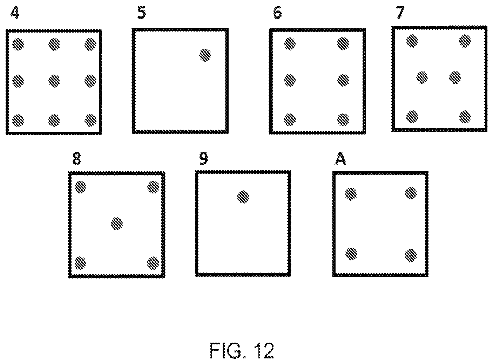

As the oil-water emulsion passed by the transducer, the trapping lines of oil droplets were observed and characterized. The characterization involved the observation and pattern of the number of trapping lines across the fluid channel, as shown in **Figure 12,** for seven of the ten resonance frequencies identified in **Figure 11.** Different displacement profiles of the transducer can produce different (more) trapping lines in the standing waves, with more gradients in displacement profile generally creating higher trapping forces and more trapping lines.

**Figure 13** is a numerical model showing a pressure field that matches the 9 trapping line pattern. The numerical model is a two-dimensional model; and therefore only three trapping lines are observed. Two more sets of three trapping lines exist in the third dimension perpendicular to the plane of the page.

In the present systems, the system is operated at a voltage such that the particles (i.e. biomolecules or cells) are trapped in the ultrasonic standing wave, i.e., remain in a stationary position. The particles are collected in along well defined trapping lines, separated by half a wavelength. Within each nodal plane, the particles are trapped in the minima of the acoustic radiation potential. The axial component of the acoustic radiation force drives particles with a positive contrast factor to the pressure nodal planes, whereas particles with a negative contrast factor are driven to the pressure anti-nodal planes. The radial or lateral component of the acoustic radiation force is the force that traps the particle. It therefore must be larger than the combined effect of fluid drag force and gravitational force. In systems using typical transducers, the radial or lateral component of the acoustic radiation force is typically several orders of magnitude smaller than the axial component of the acoustic radiation force. However, the lateral force generated by the transducers of the present disclosure can be significant, on the same order of magnitude as the axial force component, and is sufficient to overcome the fluid drag force at linear velocities of up to 1 cm/s.

The lateral force can be increased by driving the transducer in higher order mode shapes, as opposed to a form of vibration where the crystal effectively moves as a piston having a uniform displacement. The acoustic pressure is proportional to the driving voltage of the transducer. The electrical power is proportional to the square of the voltage. The transducer is typically a thin piezoelectric plate, with electric field in the z-axis and primary displacement in the z-axis. The transducer is typically coupled on one side by air (i.e. the air gap within the transducer) and on the other side by the fluid of the cell culture media. The types of waves generated in the plate are known as composite waves. A subset of composite waves in the piezoelectric plate is similar to leaky symmetric (also referred to as compressional or extensional) Lamb waves. The piezoelectric nature of the plate typically results in the excitation of symmetric Lamb waves. The waves are leaky because they radiate into the water layer, which result in the generation of the acoustic standing waves in the water layer. Lamb waves exist in thin plates of infinite extent with stress free conditions on its surfaces. Because the transducers of this embodiment are finite in nature, the actual modal displacements are more complicated.

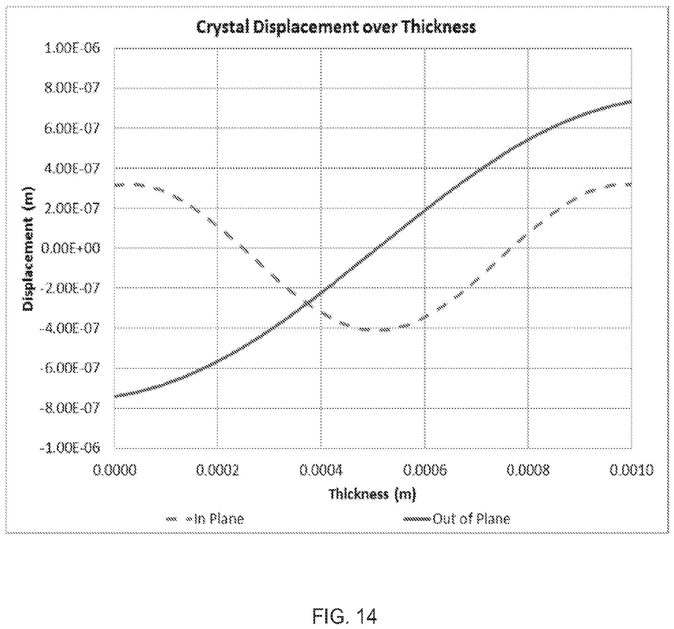

**Figure 14** shows the typical variation of the in-plane displacement (x-displacement) and out-of-plane displacement (y-displacement) across the thickness of the plate, the in-plane displacement being an even function across the thickness of the plate and the out-of-plane displacement being an odd function. Because of the finite size of the plate, the displacement components vary across the width and length of the plate. In general, a (m,n) mode is a displacement mode of the transducer in which there are m undulations in transducer displacement in the width direction and n undulations in the length direction, and with the thickness variation as described in **Figure 14.** The maximum number of m and n is a function of the dimension of the crystal and the frequency of excitation.

The transducers are driven so that the piezoelectric crystal vibrates in higher order modes of the general formula (m, n), where m and n are independently 1 or greater. Generally, the transducers will vibrate in higher order modes than (2,2). Higher order modes will produce more nodes and antinodes, result in three-dimensional standing waves in the water layer, characterized by strong gradients in the acoustic field in all directions, not only in the direction of the standing waves, but also in the lateral directions. As a consequence, the acoustic gradients result in stronger trapping forces in the lateral direction.

In embodiments, the pulsed voltage signal driving the transducer can have a sinusoidal, square, sawtooth, or triangle waveform; and have a frequency of 500 kHz to 10 MHz. The pulsed voltage signal can be driven with pulse width modulation, which produces any desired waveform. The pulsed voltage signal can also have amplitude or frequency modulation start/stop capability to eliminate streaming.

The transducer(s) is/are used to create a pressure field that generates forces of the same order of magnitude both orthogonal to the standing wave direction and in the standing wave direction. When the forces are roughly the same order of magnitude, particles of size 0.1 microns to 300 microns will be moved more effectively towards regions of agglomeration ("trapping lines"). Because of the equally large gradients in the orthogonal acoustophoretic force component, there are "hot spots" or particle collection regions that are not located in the regular locations in the standing wave direction between the transducer and the reflector. Hot spots are located in the maxima or minima of acoustic radiation potential. Such hot spots represent particle collection locations which allow for better wave transmission between the transducer and the reflector during collection and stronger inter-particle forces, leading to faster and better particle agglomeration.

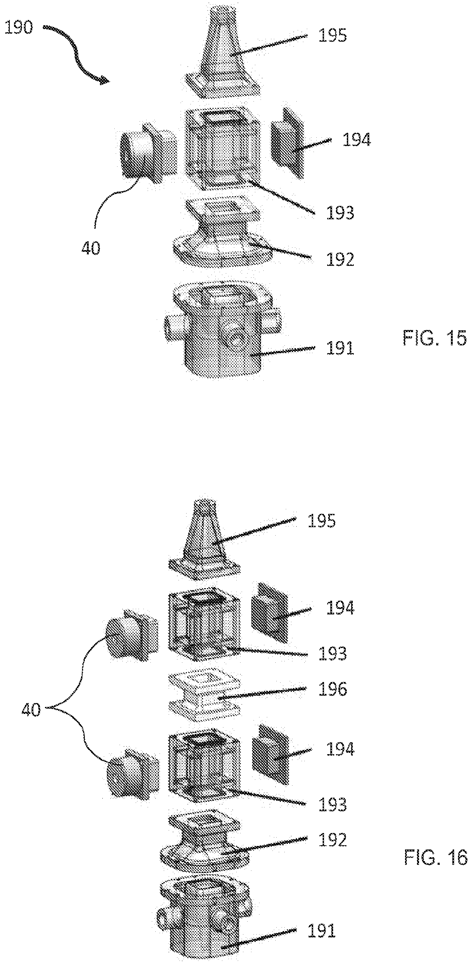

**Figure 15** and **Figure 16** are exploded views showing the various parts of acoustophoretic separators. **Figure 15** has only one separation chamber, while **Figure 16** has two separation chambers.

Referring to **Figure 15**, fluid enters the separator **190** through a four-port inlet **191**. A transition piece **192** is provided to create plug flow through the separation chamber **193.** A transducer **40** and a reflector **194** are located on opposite walls of the separation chamber. Fluid then exits the separation chamber **193** and the separator through outlet **195.**

**Figure 16** has two separation chambers **193.** A system coupler **196** is placed between the two chambers **193** to join them together.

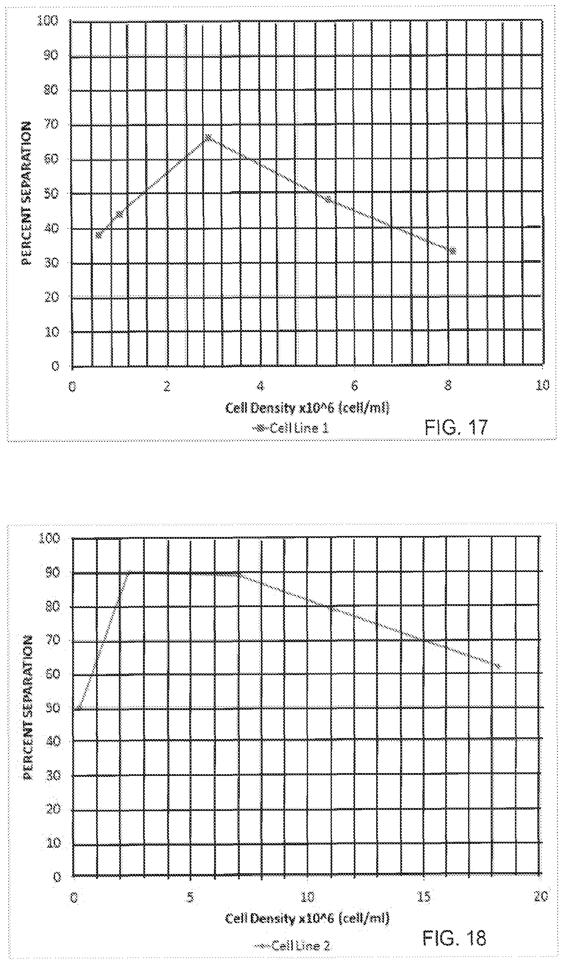

Acoustophoretic separation has been tested on different lines of Chinese hamster ovary (CHO) cells. In one experiment, a solution with a starting cell density of $8.09 \times 10^6$ cells/mL, a turbidity of 1,232 NTU, and cell viability of roughly 75% was separated using a system as depicted in **Figure 15**. The transducers were 2 MHz crystals, run at approximately 2.23 MHz, drawing 24-28 Watts. A flow rate of 25 mL/min was used. The result of this experiment is shown in **Figure 17.**

In another experiment, a solution with a starting cell density of 8.09x10$^6$ cells/mL, a turbidity of 1,232 NTU, and cell viability of roughly 75% was separated. This CHO cell line had a bi-modal particle size distribution (at size 12 μm and 20 μm). The result is shown in **Figure 18.**

**Figure 17** and **Figure 18** were produced by a Beckman Coulter Cell Viability Analyzer. Other tests revealed that frequencies of 1 MHz and 3 MHz were not as efficient as 2 MHz at separating the cells from the fluid.

In other tests at a flow rate of 10 L/hr, 99% of cells were captured with a confirmed cell viability of more than 99%. Other tests at a flow rate of 50 mL/min (i.e. 3 L/hr) obtained a final cell density of $3 \times 10^6$ cells/mL with a viability of nearly 100% and little to no temperature rise. In yet other tests, a 95% reduction in turbidity was obtained at a flow rate of 6 L/hr.

Further testing was performed using yeast as a simulant for CHO for the biological applications. For these tests, at a flow rate of 15 L/hr, various frequencies were tested as well as power levels. Table 1 shows the results of the testing.

Table 1: 2.5" x 4" System results at 15 L/hr Flow rate

| Frequency (MHz) | 30 Watts | 37 Watts | 45 Watts |
|---|---|---|---|
| 2.2211 | 93.9 | 81.4 | 84.0 |
| 2.2283 | 85.5 | 78.7 | 85.4 |
| 2.2356 | 89.1 | 85.8 | 81.0 |
| 2.243 | 86.7 | - | 79.6 |

In biological applications, it is contemplated that all of the parts of the system (e.g. the flow chamber, tubing leading to and from the bioreactor or filtering device, the sleeve containing the ultrasonic transducer and the reflector, the temperature-regulating jacket, etc.) can be separated from each other and be disposable. Avoiding centrifuges and filters allows better separation of the CHO cells without lowering the viability of the cells. The transducers may also be driven to create rapid pressure changes to prevent or clear blockages due to agglomeration of CHO cells. The frequency of the transducers may also be varied to obtain optimal effectiveness for a given power.

**Claims**

1. A system set up for use as a perfusion reactor comprising:

   A) a bioreactor (202) including a reaction vessel (220) having an internal volume, an agitator (225) for circulating a fluid medium comprising desired product and cells within the internal volume, a feed inlet (222), and an outlet (224); and
   B) an acoustophoretic filtering device (230) fluidly connected to and downstream of the bioreactor (202), the acoustophoretic filtering device comprising:

      1) an inlet (232) fluidly connected to the bioreactor outlet (242) for continuously receiving fluid medium from the bioreactor (202);
      2) a flow chamber (410) through which the fluid medium can flow; and
      3) a sleeve (420) surrounding the flow chamber (410), the sleeve (420) including an ultrasonic transducer (422) and a reflector (424) located opposite the ultrasonic transducer (422), the ultrasonic transducer (422) being driven to produce a three-dimensional acoustic standing wave in the flow chamber (410) that separates the desired product from the cells; and
      4) a product outlet (234) for removing a concentrated flow of the desired product;
      5) a recycle outlet (236) for sending the fluid medium back to the bioreactor (202), the recycle outlet (236) located downstream of the flow chamber (410) and connected to a recycle inlet (226) of the reaction vessel (220)

   wherein the acoustophoretic filtering device (230) is configured so that the three-dimensional acoustic standing wave has an axial force component and a lateral force component which are of the same order of magnitude and results in an acoustic radiation force that is larger than the combined effects of fluid drag and buoyancy or gravity, and is therefore able to trap the cells to allow more time for the acoustic wave to increase particle concentration, agglomeration and/or coalescence.

**2.** The system of claim 1, wherein the bioreactor is a perfusion bioreactor.

**3.** The system of claim 1, wherein the sleeve (420) is separable from the flow chamber.

**4.** The system of claim 1, wherein the filtering device (230) further comprises a jacket (430) located between the sleeve (420) and the flow chamber (410), the jacket (430) being used to regulate the temperature of the fluid medium in the flow chamber (410).

**5.** The system of claim 3, wherein the jacket (430), the sleeve (420), and the flow chamber (410) are separable from each other and are disposable.

**6.** The system of claim 1, wherein the ultrasonic transducer (410) comprises a piezoelectric material that can vibrate in a higher order mode shape.

**7.** The system of claim 1, wherein the ultrasonic transducer (410) comprises:

a housing having a top end, a bottom end, and an interior volume; and
a piezoelectrical material at the bottom end of the housing having an exposed exterior surface and an interior surface, the piezoeletrical material being able to vibrate when driven by a signal; and
an air gap between the piezoelectric material and the top end of the housing.

**8.** The system of claim 6, wherein a backing layer contacts the interior surface of the piezoelectric material, the backing layer being made of a substantially acoustically transparent material.

**9.** The system of claim 8, wherein the substantially acoustically transparent material is balsa wood, cork, or foam; or wherein the substantially acoustically transparent material has a thickness of up to 2.5 cm (1 inch), or wherein the substantially acoustically transparent material is in the form of a lattice.

**10.** The system of claim 6, wherein an exterior surface of the piezoelectric material is covered by a wear surface material with a thickness of a half wavelength or less, the wear surface material being a urethane, epoxy, or silicone coating.

**11.** The system of claim 6, wherein the piezoelectric material has no backing layer or wear layer.

**12.** The system of claim 1, wherein the reflector has a non-planar surface.

**13.** A method for separating desired product from a fluid medium comprising desired product, cells and fluid, the method comprising:

culturing the fluid medium in a perfusion bioreactor (202) including a reaction vessel (220) having an internal volume, an agitator (225) for circulating an fluid medium comprising desired product and cells within the internal volume, a feed inlet (222), and an outlet (224); and
flowing at least a portion of the fluid medium from the perfusion bioreactor (202) to an acoustophoretic filtering device (230) that comprises:

1) an inlet (232) fluidly connected to the perfusion bioreactor outlet (242) for continuously receiving fluid medium from the perfusion bioreactor (202) ;
2) a flow chamber (410) through which the fluid medium can flow;
3) a sleeve (420) surrounding the flow chamber (410), the sleeve (420) including an ultrasonic transducer (422) and a reflector (424) located opposite the ultrasonic transducer (422);
4) a product outlet (234) for removing a concentrated flow of the desired product; and
5) a recycle outlet (236) for sending the fluid medium back to the perfusion bioreactor (202), the recycle outlet (236) downstream of the flow chamber (410) and connected to a recycle inlet (226) of the reaction vessel (220)

driving the ultrasonic transducer (422) to produce a three-dimensional standing wave in the flow chamber (410) that separates the desired product from the cells, wherein the three-dimensional acoustic standing wave has an axial force component and a lateral force component which are of the same order of magnitude and results in an acoustic radiation force that is larger than the combined effects of fluid drag and buoyancy or gravity, and is therefore able to trap the cells to allow more time for the acoustic wave to increase particle concentration, agglomeration and/or coalescence; and

recovering a concentrated flow of the desired product through the product outlet (234) of the acoustophoretic filtering device (230);

sending the fluid medium back to the perfusion bioreactor (202) through the recycle outlet (236) of the acoustophoretic filtering device (230); and

further processing the concentrated flow of the desired product,

**14.** The method of claim 13, wherein the desired product is recombinant proteins or monoclonal antibodies.

**15.** The method of claim 13, wherein the further processing is adapted to divert and collect the desired product for further processing.

**16.** The method of claim 15, wherein the further processing is adapted to isolate or recover the biomolecules from the concentrated flow.


**Patentansprüche**

**1.** System, das zur Verwendung als Perfusionsreaktor eingerichtet ist, umfassend:

A) einen Bioreaktor (202, 610), der ein Reaktionsgefäß (220), welches ein Innenvolumen aufweist, einen Rührer (225) zum Zirkulieren eines Fluidmediums, das das gewünschte Produkt und Zellen innerhalb des Innenvolumens umfasst, einen Zufuhreinlass (222) und einen Auslass (224) umfasst; und
B) eine akustophoretische Filtervorrichtung (230, 630), die mit dem Bioreaktor (202) in Fluidverbindung steht und diesem nachgelagert ist, wobei die akustophoretische Filtervorrichtung umfasst:

1) einen Einlass (232), der in Fluidverbindung mit dem Bioreaktorauslass (242) steht, um kontinuierlich Fluidmedium aus dem Bioreaktor (202) aufzunehmen;
2) eine Strömungskammer (410), durch die das Fluidmedium strömen kann; und
3) eine Hülse (420), die die Strömungskammer (410) umgibt, wobei die Hülse (420) einen Ultraschallwandler (422) und einen Reflektor (424) aufweist, der gegenüber dem Ultraschallwandler (422) angeordnet ist, wobei der Ultraschallwandler (422) angetrieben wird, um eine dreidimensionale akustische stehende Welle in der Strömungskammer (410) zu erzeugen, die das gewünschte Produkt von den Zellen trennt;
4) einen Produktauslass (234) zum Entfernen eines konzentrierten Stroms des gewünschten Produkts; und
5) einen Rezirkulationsauslass (236) zum Zurückleiten des Fluidmediums zu dem Bioreaktor (202), wobei der Rezirkulationsauslass (236) der Strömungskammer (410) nachgeordnet ist und mit einem Rezirkulationseinlass (226) des Reaktionsgefäßes (220) verbunden ist;

wobei die akoustophoretische Filtervorrichtung (230) so eingerichtet ist, dass die dreidimensionale akustische stehende Welle eine axiale Kraftkomponente und eine laterale Kraftkomponente aufweist, die in der gleichen Größenordnung liegen, und zu einer akustischen Abstrahlungskraft führt, die größer ist als die kombinierten Effekte von Fluidwiderstand und Auftrieb oder Schwerkraft, und daher in der Lage ist, die Zellen einzufangen, um der akustischen Welle mehr Zeit zu geben, um die Partikelkonzentration, Agglomeration und/oder Koaleszenz zu erhöhen.

**2.** System nach Anspruch 1, wobei der Bioreaktor ein Perfusions-Bioreaktor ist.

**3.** System nach Anspruch 1, wobei die Hülse (420) von der Strömungskammer trennbar ist.

**4.** System nach Anspruch 1, wobei die Filtervorrichtung (230) des Weiteren einen Mantel (430) umfasst, der zwischen der Hülse (420) und der Strömungskammer (410) angeordnet ist, wobei der Mantel (430) dazu verwendet wird, die Temperatur des Fluidmediums in der Strömungskammer (410) zu regulieren.

**5.** System nach Anspruch 3, wobei der Mantel (430), die Hülse (420) und die Strömungskammer (410) voneinander trennbar sind und entsorgbar sind.

**6.** System nach Anspruch 1, wobei der Ultraschallwandler (410) ein piezoelektrisches Material umfasst, das in einer Modenform höherer Ordnung schwingen kann.

7. System nach Anspruch 1, wobei der Ultraschallwandler (410) umfasst:

   ein Gehäuse, das ein oberes Ende, ein unteres Ende und ein Innenvolumen aufweist; und
   ein piezoelektrisches Material an dem unteren Ende des Gehäuses, das eine freiliegende Außenfläche und eine Innenfläche aufweist, wobei das piezoelektrische Material in der Lage ist, zu schwingen, wenn es durch ein Signal angetrieben wird; und
   einen Luftspalt zwischen dem piezoelektrischen Material und dem oberen Ende des Gehäuses.

8. System nach Anspruch 6, wobei eine Trägerschicht die Innenfläche des piezoelektrischen Materials berührt, wobei die Trägerschicht aus einem im Wesentlichen akustisch transparenten Material hergestellt ist.

9. System nach Anspruch 8, wobei das im Wesentlichen akustisch transparente Material Balsaholz, Kork oder Schaumstoff ist; oder
   wobei das im Wesentlichen akustisch transparente Material eine Dicke von bis zu 2,5 cm (1 Zoll) aufweist, oder
   wobei das im Wesentlichen akustisch transparente Material in Form eines Gitters vorliegt.

10. System nach Anspruch 6, wobei eine Außenfläche des piezoelektrischen Materials mit einem Verschleißflächenmaterial mit einer Dicke von einer halben Wellenlänge oder weniger bedeckt ist, wobei das Verschleißflächenmaterial eine Urethan-, Epoxid- oder Silikonbeschichtung ist.

11. System nach Anspruch 6, wobei das piezoelektrische Material keine Trägerschicht oder Verschleißschicht aufweist.

12. System nach Anspruch 1, wobei der Reflektor eine nichtplanare Oberfläche aufweist.

13. Verfahren zum Abtrennen eines gewünschten Produkts von einem Fluidmedium, das das gewünschte Produkt, Zellen und Fluid umfasst, wobei das Verfahren umfasst:

    Kultivieren des Fluidmediums in einem Perfusions-Bioreaktor (202), der ein Reaktionsgefäß (220), das ein Innenvolumen aufweist, einen Rührer (225) zum Zirkulieren eines Fluidmediums, das das gewünschte Produkt und Zellen in dem Innenvolumen umfasst, einen Zuführeinlass (222) und einen Auslass (224) umfasst; und
    Durchleiten zumindest eines Teils des Fluidmediums von dem Perfusions-Bioreaktor (202) zu einer akustophoretischen Filtervorrichtung (230), die umfasst:

    1) einen Einlass (232), der in Fluidverbindung mit dem Bioreaktorauslass (242) steht, um kontinuierlich Fluidmedium aus dem Bioreaktor (202) aufzunehmen;
    2) eine Strömungskammer (410), durch die das Fluidmedium strömen kann; und
    3) eine Hülse (420), die die Strömungskammer (410) umgibt, wobei die Hülse (420) einen Ultraschallwandler (422) und einen Reflektor (424) aufweist, der gegenüber dem Ultraschallwandler (422) angeordnet ist;
    4) einen Produktauslass (234) zum Entfernen eines konzentrierten Stroms des gewünschten Produkts; und
    5) einen Rezirkulationsauslass (236) zum Zurückleiten des Fluidmediums zu dem Perfusions-Bioreaktor (202), wobei der Rezirkulationsauslass (236) der Strömungskammer (410) nachgeordnet ist und mit einem Rezirkulationseinlass (226) des Reaktionsgefäßes (220) verbunden ist;

    Antreiben des Ultraschallwandlers (422), um eine dreidimensionale akustische stehende Welle in der Strömungskammer (410) zu erzeugen, die das gewünschte Produkt von den Zellen trennt, wobei die dreidimensionale akustische stehende Welle eine axiale Kraftkomponente und eine laterale Kraftkomponente aufweist, die in der gleichen Größenordnung liegen, und die zu einer akustischen Abstrahlungskraft führt, die größer ist als die kombinierten Effekte von Fluidwiderstand und Auftrieb oder Schwerkraft, und die daher in der Lage ist, die Zellen einzufangen, um der akustischen Welle mehr Zeit zu geben, um die Partikelkonzentration, Agglomeration und/oder Koaleszenz zu erhöhen; und
    Wiedergewinnen eines konzentrierten Durchflusses des gewünschten Produkts durch den Produktauslass (234) der akustophoretischen Filtervorrichtung (230);
    Zurückleiten des flüssigen Mediums zu dem Perfusions-Bioreaktor (202) durch den Rezirkulationsauslass (236) der akustophoretischen Filtervorrichtung (230); und
    weiteres Verarbeiten des konzentrierten Durchflusses des gewünschten Produkts.

14. Verfahren nach Anspruch 13, wobei das gewünschte Produkt rekombinante Proteine oder monoklonale Antikörper sind.

**15.** Verfahren nach Anspruch 13, wobei die weitere Verarbeitung geeignet ist, das gewünschte Produkt zur weiteren Verarbeitung auszuleiten und zu sammeln.

**16.** Verfahren nach Anspruch 15, wobei die weitere Verarbeitung geeignet ist, die Biomoleküle aus dem konzentrierten Strom zu isolieren oder zurückzugewinnen.

## Revendications

**1.** Système destiné à être utilisé en tant que réacteur à perfusion comprenant :

A) un bioréacteur (202, 610) comprenant une cuve de réaction (220) présentant un volume intérieur, un agitateur (225) pour faire circuler un milieu fluide comprenant un produit souhaité et des cellules dans le volume intérieur, une entrée d'alimentation (222), et une sortie (224) ; et

B) un dispositif de filtrage acoustophorétique (230, 630) connecté de manière fluidique au bioréacteur (202), et en aval de celui-ci, le dispositif de filtrage acoustophorétique comprenant :

1) une entrée (232) connectée de manière fluidique à la sortie du bioréacteur (242) afin de recevoir de manière continue le milieu fluide en provenance du bioréacteur (202) ;

2) une chambre d'écoulement (410) à travers laquelle peut s'écouler le milieu fluide ; et

3) un manchon (420) entourant la chambre d'écoulement (410), le manchon (420) comprenant un transducteur ultrasonore (422) et un réflecteur (424) situé à l'opposé du transducteur ultrasonore (422), le transducteur ultrasonore (422) étant commandé afin de produire une onde stationnaire acoustique tridimensionnelle dans la chambre d'écoulement (410), qui sépare le produit souhaité des cellules ; et

4) une sortie de produit (234) pour retirer un flux concentré du produit souhaité ;

5) une sortie de recyclage (236) pour renvoyer le milieu fluide vers le bioréacteur (202), la sortie de recyclage (236) étant située en aval de la chambre d'écoulement (410), et connectée à une entrée de recyclage (226) de la cuve de réaction (220)

Dans lequel le dispositif de filtrage acoustophorétique (230) est configuré de telle sorte que l'onde stationnaire acoustique tridimensionnelle présente une composante de force axiale et une composante de force latérale qui sont du même ordre de grandeur, et résulte en une force de rayonnement acoustique qui est plus grande que les effets combinés de la traînée et de la flottabilité ou de la pesanteur du fluide, et peut donc piéger les cellules afin de laisser plus de temps à l'onde acoustique pour accroître la concentration, l'agglomération et / ou la coalescence des particules.

**2.** Système selon la revendication 1, dans lequel le bioréacteur est un bioréacteur à perfusion.

**3.** Système selon la revendication 1, dans lequel le manchon (420) peut être séparé de la chambre d'écoulement.

**4.** Système selon la revendication 1, dans lequel le dispositif de filtrage (230) comprend en outre une chemise (430) située entre le manchon (420) et la chambre d'écoulement (410), la chemise (430) étant utilisée pour réguler la température du milieu fluide dans la chambre d'écoulement (410).

**5.** Système selon la revendication 3, dans lequel la chemise (430), le manchon (420), et la chambre d'écoulement (410) peuvent être séparés les uns des autres, et sont jetables.

**6.** Système selon la revendication 1, dans lequel le transducteur ultrasonore (410) comprend un matériau piézoélectrique qui peut vibrer dans une forme d'un mode d'ordre supérieur.

**7.** Système selon la revendication 1, dans lequel le transducteur ultrasonore (410) comprend :

un logement présentant une extrémité supérieure, une extrémité inférieure, et un volume intérieur ; et
un matériau piézoélectrique à l'extrémité inférieure du logement présentant une surface extérieure exposée et une surface intérieure, le matériau piézoélectrique pouvant vibrer quand il est commandé par un signal ; et
un intervalle d'air entre le matériau piézoélectrique et l'extrémité supérieure du logement.

**8.** Système selon la revendication 6, dans lequel une couche de support est en contact avec la surface intérieure du matériau piézoélectrique, la couche de support étant réalisée dans un matériau sensiblement transparent acoustiquement.

9. Système selon la revendication 8,
dans lequel le matériau sensiblement transparent acoustiquement est du balsa, du liège, ou une mousse ; ou
dans lequel le matériau sensiblement transparent acoustiquement présente une épaisseur jusqu'à 2,5 cm (1 pouce), ou
dans lequel le matériau sensiblement transparent acoustiquement se présente sous une forme de treillis.

10. Système selon la revendication 6, dans lequel une surface extérieure du matériau piézoélectrique est couverte par un matériau de surface d'usure présentant une épaisseur égale ou inférieure à une demi-longueur d'onde, le matériau de surface d'usure étant un revêtement d'uréthane, d'époxyde, ou de silicone.

11. Système selon la revendication 6, dans lequel le matériau piézoélectrique ne présente aucune couche de support ni aucune couche d'usure.

12. Système selon la revendication 1, dans lequel le réflecteur a une surface non plane.

13. Procédé destiné à séparer un produit souhaité d'un milieu fluide comprenant le produit souhaité, des cellules et un fluide, le procédé comprenant les étapes suivantes :

mettre en culture le milieu fluide dans un bioréacteur à perfusion (202) comprenant une cuve de réaction (220) présentant un volume intérieur, un agitateur (225) pour faire circuler un milieu fluide comprenant un produit souhaité et des cellules dans le volume intérieur, une entrée d'alimentation (222), et une sortie (224) ; et
faire s'écouler au moins une partie du milieu fluide à partir du bioréacteur à perfusion (202) dans un dispositif de filtrage acoustophorétique (230) comprenant :

1) une entrée (232) connectée de manière fluidique à la sortie du bioréacteur à perfusion (242) afin de recevoir de manière continue le milieu fluide en provenance du bioréacteur à perfusion (202) ;
2) une chambre d'écoulement (410) à travers laquelle le milieu fluide peut s'écouler ;
3) un manchon (420) entourant la chambre d'écoulement (410), le manchon (420) comprenant un transducteur ultrasonore (422) et un réflecteur (424) situé à l'opposé du transducteur ultrasonore (422) ;
4) une sortie de produit (234) pour retirer un flux concentré du produit souhaité ; et
5) une sortie de recyclage (236) pour renvoyer le milieu fluide vers le bioréacteur à perfusion (202), la sortie de recyclage (236) étant située en aval de la chambre d'écoulement (410), et connectée à une entrée de recyclage (226) de la cuve de réaction (220)

commander le transducteur ultrasonore (422) afin de produire une onde stationnaire tridimensionnelle dans la chambre d'écoulement (410) séparant le produit souhaité des cellules, dans lequel l'onde stationnaire acoustique tridimensionnelle présente une composante de force axiale et une composante de force latérale qui sont du même ordre de grandeur et résulte en une force de rayonnement acoustique plus grande que les effets combinés de la traînée et de la flottabilité ou de la pesanteur du fluide, et est donc capable de piéger les cellules afin de laisser plus de temps à l'onde acoustique pour accroître la concentration, l'agglomération et / ou la coalescence des particules ; et
récupérer un flux concentré du produit souhaité à travers la sortie de produit (234) du dispositif de filtrage acoustophorétique (230) ;
renvoyer le milieu fluide vers le bioréacteur à perfusion (202) à travers la sortie de recyclage (236) du dispositif de filtrage acoustophorétique (230) ; et
traiter ultérieurement le flux concentré du produit souhaité.

14. Procédé selon la revendication 13, dans lequel le produit souhaité est des protéines recombinées ou des anticorps monoclonaux.

15. Procédé selon la revendication 13, dans lequel le traitement ultérieur est adapté pour détourner et recueillir le produit souhaité pour un traitement ultérieur.

16. Procédé selon la revendication 15, dans lequel le traitement ultérieur est adapté pour isoler ou récupérer les biomolécules du flux concentré.

FIG. 1

FIG. 3

FIG. 2

FIG. 4

FIG. 5

FIG. 6

62

60 HOUSING

61 POSITIVE ELECTRODE

58 BACKING LAYER

56 EPOXY LAYER

54 CERAMIC CRYSTAL

52 EPOXY LAYER

65 ELECTRICAL CONNECTION

63 NEGATIVE ELECTRODE

50 WEAR PLATE

FIG. 7

FIG. 8

FIG. 9

FIG. 10

EP 2 953 700 B1

FIG. 11

FIG. 12

FIG. 13

FIG. 14

FIG. 15

FIG. 16

28

FIG. 17

FIG. 18

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2011123392 A1

**Non-patent literature cited in the description**

- Ultrasonic separation of suspended particles. *2001 IEEE ULTRASONICS SYMPOSIUM PROCEEDINGS,* 07 October 2001, 649-659